(19) Europäisches Patentamt
European Patent Office
Office européen des brevets

(11) EP 2 334 284 B1

(12) EUROPÄISCHE PATENTSCHRIFT

(45) Veröffentlichungstag und Bekanntmachung des Hinweises auf die Patenterteilung:
**08.08.2012 Patentblatt 2012/32**

(21) Anmeldenummer: **09778701.4**

(22) Anmeldetag: **24.09.2009**

(51) Int Cl.:
***A61K 9/16*** *(2006.01)* ***A61K 9/20*** *(2006.01)*
***A61K 31/137*** *(2006.01)*

(86) Internationale Anmeldenummer:
**PCT/EP2009/006907**

(87) Internationale Veröffentlichungsnummer:
**WO 2010/034497 (01.04.2010 Gazette 2010/13)**

# (54) KOMPAKTIERTES CINACALCET

COMPACTED CINACALCET

CINACALCET COMPACTÉ

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **25.09.2008 DE 102008048836**

(43) Veröffentlichungstag der Anmeldung:
**22.06.2011 Patentblatt 2011/25**

(73) Patentinhaber: **Ratiopharm GmbH**
**89079 Ulm (DE)**

(72) Erfinder:
- **RIMKUS, Katrin**
  **80798 München (DE)**
- **MUSKULUS, Frank**
  **88471 Laupheim (DE)**
- **BRUECK, Sandra**
  **85570 Ottenhofen (DE)**
- **PAETZ, Jana**
  **53177 Bonn (DE)**

(74) Vertreter: **Aechter, Bernd**
**Patentanwälte/European Patent Attorneys**
**Ter Meer Steinmeister & Partner GbR**
**Mauerkircherstrasse 45**
**81679 München (DE)**

(56) Entgegenhaltungen:
**WO-A1-2005/034928** **WO-A2-2008/027522**
**WO-A2-2008/064202** **US-A1- 2007 185 211**

EP 2 334 284 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Intermediat, erhältlich durch gemeinsames Kompaktieren von (i) kristallinem Cinacalcet oder eines pharmazeutisch verträglichen Salzes davon, mit (ii) einem Hydrophilierungsmittel sowie Tabletten enthaltend die erfindungsgemäßen Intermediate. Die Erfindung betrifft ferner Cinacalcet-Tabletten mit bimodaler Porengrößenverteilung und ein Verfahren zur Herstellung der erfindungsgemäßen Tabletten. Schließlich betrifft die Erfindung die Verwendung eines pH-Anpassers zur Herstellung von Cinacalcet-Formulierungen, die vorzugsweise unabhängig von den Mahlzeiten verabreicht werden können.

N-((1R)-1-(1-naphthyl)ethyl]-3-[3-(trifluormethyl)phenyl]propan-1-amin ist unter dem INN-Namen "Cinacalcet" bekannt und weist folgende Strukturformel auf:

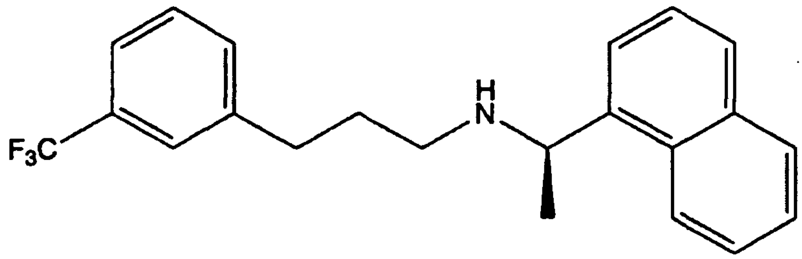

Das Calcimimetikum Cinacalcet dient zur Behandlung des sekundären Hyperparathyreoidismus infolge einer chronischen Niereninsuffizienz. Zudem ist die Substanz zur Behandlung der Hypercalcämie bei Patienten mit Nebenschilddrüsenkarzinom zugelassen.

Synthese und Wirkung von Cinacalcet sind in EP 1 203 761 B1 beschrieben. Patienten mit einer chronischen Nierenerkrankung bekommen infolge ihrer Grunderkrankung häufig eine Nebenschilddrüsen-Überfunktion (sekundären Hyperparathyreoidismus). Insuffiziente Nieren scheiden mit dem Urin weniger Phosphat aus und bilden weniger aktives Vitamin D3, das für die Aufrechterhaltung eines physiologischen Calciumionen-Blutspiegels notwendig ist. Wenn der Calciumionenspiegel sinkt, wird in den Nebenschilddrüsen vermehrt Parathormon ausgeschüttet. Die Parathormon-Überproduktion bewirkt wiederum, dass Calciumionen aus den Knochen mobilisiert wird und die Knochen brüchiger werden. Cinacalcet bindet an die calciumempfindlichen Rezeptoren an der Oberfläche der Nebenschilddrüsenzellen. Dadurch wird die Empfindlichkeit des Rezeptors gegenüber extrazellulären Calciumlonen gesteigert und ein höherer Calciumspiegel im Blut als tatsächlich vorhanden simuliert. Als Folge sinkt die Parathormon-Sekretion, damit wird weniger Calcium aus dem Knochen freigesetzt.

US 2007/185211 A1 bezieht sich auf neue kristalline Cinacalecthydrochlorid-Strukturen und deren Solvate, die durch Röntgenkristallographie bestimmt werden. Des Weiteren wird eine pharmazeutische Zusammensetzung bestimmter Cinacalecthydrochlorid-Strukturen beschrieben, welche noch zusätzlich wenigstens einen pharmazeutisch akzeptablen Hilfsstoff enthalten.

WO 2008/027522 A2 beschreibt Zusammensetzungen einer festen Form von Cinacalcet in Verbindung mit einer Trägerkomponente. Das Cinacalcet liegt hierbei nicht in partikulärer und nicht in kristalliner Form vor. Es liegt hingegen in Form einer festen Lösung vor.

Cinacalcet ist durch Sprühtrocknung auch in amorpher Form erhältlich, siehe WO 2008/000422 A1. Wirkstoffe in amorpher Form zeigen jedoch häufig nachteilige Eigenschaften im Hinblick auf die Lagerstabilität.

Die WO 2008/064202 beschreibt Cinacalcet-haltige Zusammensetzungen mit verzögerter Freisetzung. Darreichungsformen mit verzögerter Freisetzung werden üblicherweise für spezielle Anwendungen verwendet. Für eine Vielzahl von Anwendungen sind jedoch Darreichungsformen mit sofortiger Freisetzung wünschenswert.

Die derzeitig vermarkteten Filmtabletten sind Tabletten mit sofortiger Freisetzung (= Immediate-Release Tabletten) und in WO 2005/034928 beschrieben. Die Tabletten enthalten Cinacalcet in mikronisierter Form mit einem Wirkstoffanteil von ungefähr 18 %. Dabei sollten die Filmtabletten mit oder kurz nach einer Mahlzeit eingenommen werden, da sich die Bioverfügbarkeit bei gleichzeitiger Nahrungsaufnahme um 50 bis 80 Prozent erhöht und erst dann akzeptabel ist.

Mit der Mikronisierung von Cinacalcet gehen jedoch einige Nachteile einher. Zunächst resultiert die Mikronisierung in einem Wirkstoff mit unerwünscht niedriger Fließfähigkeit. Ferner ist der mikronisierte Wirkstoff schwieriger zu verpressen, gelegentlich kommt es zu ungleichmäßiger Wirkstoffverteilung innerhalb der zu verpressenden pharmazeutischen Formulierung. Durch die starke Vergrößerung der Oberfläche während der Mikronisierung nimmt zudem die Oxidationsempfindlichkeit des Wirkstoffs zu.

Aufgabe der vorliegenden Erfindung war es daher, die vorstehend genannten Nachteile zu überwinden. Es soll der Wirkstoff in einer Form bereitgestellt werden, die eine gute Fließfähigkeit aufweist und eine gute Verpressung

ermöglicht. Ferner soll eine gleichmäßige Verteilung des Wirkstoffs gewährleistet sein. Eine Mikronisierung des Wirkstoffs soll vermieden werden.

Weiterhin soll der Wirkstoff in einer Form bereitgestellt werden, die gute Löslichkeit bei zeitgleich guter Lagerstabilität gewährleistet. Auch nach 2-jähriger Lagerung (bzw. 3-monatiger Lagerung unter Stressbedingungen) soll eine entsprechend gute Löslichkeit erzielbar sein. Es soll eine Verabreichung unabhängig von den Mahlzeiten ermöglicht werden. Insbesondere soll eine Löslichkeit von größer 3 mg/ml, insbesondere von 10 mg/ml erreicht werden. Ferner soll eine Lagerstabilität von 12 Monaten bei 40 °C und 75 % Luftfeuchte erzielt werden. Die Verunreinigungen sollen nach derartiger Lagerung < 2 Gew.-%, insbesondere < 1 Ges.-% sein. Ferner soll es möglich sein, Cinacalcet-Tabletten sowohl mit rascher Zerfallszeit als auch mit vorteilhafter Härte bereit zu stellen.

Alle vorstehend genannten vorteilhaften Eigenschaften sollen zudem bei hohem Wirkstoffanteil (z.B. bei Wirkstoffgehalten von 20 %. 30 %. 40 % und/oder 50 %) erzielbar sein. Insbesondere sollen die vorstehen genannten Eigenschaften zudem bei hohem Wirkstoffantell und zugleich hoher "Content Uniformity" erzielbar sein.

Die Aufgaben der vorliegenden Erfindung konnten durch ein Intermediat gelöst werden, das durch gemeinsames Kompaktieren von Cinacalcet und Hydrophilierungsmittel erhältlich ist, sowie durch Verwendung des Intermediats zur Herstellung von Tabletten mit sofortiger Freisetzung, wobei die Tabletten insbesondere eine bimodale Porenverteilung aufweisen.

Gegenstand der Erfindung ist somit ein Intermediat, erhältlich durch gemeinsames Kompaktieren von

(i) kristallinem Cinacalcet oder eines pharmazeutisch verträglichen Salzes davon, mit
(ii) einem Hydrophilierungsmittel.

Grundsätzlich umfasst im Rahmen dieser Anmeldung der Begriff "Cinacalcet" (i) sowohl die vorstehend dargestellte "freie Base" als auch pharmazeutisch verträgliche Salze davon. Hierbei kann es sich um ein oder mehrere Salze handeln, die auch im Gemisch vorliegen können. Unter "Salz" wird hierbei verstanden, dass die Amingruppe des Cinacalcets protoniert wurde, wobei sich ein positiv geladenes Stickstoffatom bildet, das mit einem entsprechenden Gegenanion assoziiert ist.

Bevorzugt werden als Salze Säureadditionssalze verwendet. Beispiele für geeignete Salze sind Hydrochloride, Carbonate, Hydrogencarbonate, Acetate, Lactate, Butyrate, Propionate, Sulfate, Methansulfonate, Citrate, Tartrate, Nitrate, Sulfonate, Oxalate und/oder Succinate.

Besonders bevorzugt handelt es sich im Falle von Cinacalcet bei dem pharmazeutisch verträglichen Salz um Cinacalcet-Hydrochlorid. Ebenfalls besonders bevorzugt handelt es sich bei dem pharmazeutisch verträglichen Salz um Cinacalcet-Carbonat. Weiterhin besonders bevorzugt handelt es sich bei dem pharmazeutisch verträglichen Salz um Cinacalcet-Methansulfonat.

Bei dem verwendeten Cinacalcet (i), bevorzugt bei dem verwendeten Cinacalcet-Hydrochlorid, handelt es sich üblicherweise um kristallines Material, das nicht mikronisiert wurde. Bevorzugt wird Cinacalcet-Hydrochlorid in polymorpher Form I verwendet. Die polymorphe Form I ist z.B. in WO 2007/62147 offenbart.

Der Begriff "nicht mikronisiertes Cinacalcet" bezieht sich im Rahmen dieser Erfindung auf partikuläres Cinacalcet, das im allgemeinen einen mittleren Teilchendurchmesser (D50) von 60 bis 250 $\mu$m, bevorzugt von 65 bis 200 $\mu$m, mehr bevorzugt von 70 bis 125 $\mu$m, und insbesondere von 75 bis 110 $\mu$m aufweist.

Der Ausdruck "mittlerer Teilchendurchmesser" bezieht sich im Rahmen dieser Erfindung auf den D50-Wert des volumenmittleren Teilchendurchmessers, der mittels Laserdiffraktometrie bestimmt wurde. Insbesondere wurde zur Bestimmung ein Mastersizer 2000 von Malvern Instruments verwendet. Alle Messbedingungen werden wie auf Seiten 9 und 10 von WO 2005/034928 beschrieben gewählt, d.h. Naßmessung, 1750 rpm, Span® 85 als Dispergiermittel, Auswertung gemäß der Fraunhofer Methode. Der mittlere Teilchendurchmesser, der auch als D50-Wert der integralen Volumenverteilung bezeichnet wird, wird im Rahmen dieser Erfindung als der Teilchendurchmesser definiert, bei dem 50 Volumen-% der Teilchen einen kleineren Durchmesser haben als der Durchmesser, der dem D50-Wert entspricht. Ebenso haben dann 50 Volumen-% der Teilchen einen größeren Durchmesser als der D50-Wert.

Analog wird als D10-Wert der Teilchendurchmesser definiert, bei dem 10 Volumen-% der Teilchen einen kleineren Durchmesser haben als der Durchmesser, der dem D10-Wert entspricht. Ebenso wird als D90 -Wert der Teilchendurchmesser definiert, bei dem 90 Volumen-% der Teilchen einen kleineren Durchmesser haben als der Durchmesser, der dem D90-Wert entspricht.

Das nicht mikronisierte Cinacalcet weist ferner üblicherweise D10-Werte von 1 bis 50 $\mu$m, mehr bevorzugt von 1 bis 30 $\mu$m, und insbesondere von 2 bis 25 $\mu$m auf. Weiterhin weist das nicht mikronisierte Cinacalcet weist üblicherweise D90-Werte von 200 bis 800 $\mu$m, mehr bevorzugt von 250 bis 700 $\mu$m, und insbesondere von 300 bis 600 $\mu$m auf.

Kristallines Cinacalcet liegt üblicherweise in Form von Nadeln vor. Die Charakterisierung mittels volumenmittlerem Teilchendurchmesser ist daher häufig nicht spezifisch genug.

Es hat sich gezeigt, dass eine exaktere Charakterisierung von vorteilhaft verwendbaren Cinacalcet, insbesondere Cinacalcet-Hydrochlorid, durch die Beschreibung der spezifischen Oberfläche erfolgen kann.

In einer bevorzugten Ausführungsform wird (i) kristallines Cinacalcet oder ein pharmazeutisch verträgliches Salz davon mit einer spezifischen Oberfläche von 0.01 bis 12 $m^2/g$, mehr bevorzugt von 0.1 bis 8 $m^2/g$, insbesondere von 0.1 bis 7 $m^2/g$ eingesetzt.

Die spezifische Oberfläche wird im Rahmen dieser Erfindung gemäß Gasadsorptionsmethode, insbesondere mittels BET-Methode, bestimmt.

In einer bevorzugten Ausführungsform weist das verwendete Cinacalcet (i), insbesondere das Cinacalcet-Hydrochlorid, einen Wassergehalt von 0,01 bis 0,20 Gew.-%. mehr bevorzugt von 0,02 bis 0,10 Gew.-% auf. Der Restwassergehalt wird nach der Karl Fischer Methode bestimmt, wobei ein Coulometer bei 160 °C verwendet wird. Bevorzugt wird ein Metrohm 831 KF Coulometer mit einer Titrierzelle ohne Diaphragma verwendet. Üblicherweise wird eine Probe von 20 mg Cinacalcet analysiert.

Es hat sich gezeigt, dass ein höherer Wassergehalt die Fließfähigkeit negativ beeinflussen würde und damit bei einem hohen Wirkstoffgehalt (drug load) die Gleichförmigkeit des Gehalts (Content Uniformity).

Bei dem "Hydrophilierungsmittel" (ii) handelt es sich im Rahmen dieser Erfindung im Allgemeinen um einen Stoff, welcher in der Lage ist, sich an Cinacalcet oder Salze davon (chemisch oder physikalisch) anzulagern und die Hydrophilität der Oberfläche zu erhöhen.

Bei dem Hydrophilierungsmittel (ii) kann es sich um hydrophile Polymere handeln. Darunter sind Polymere zu verstehen, die hydrophile Gruppen aufweisen. Beispiele für geeignete hydrophile Gruppen sind Hydroxy, Amino, Carboxy, Sulfonat. Ferner weist das zur Herstellung des Intermediats verwendbare hydrophile Polymer bevorzugt ein gewichtsmittleres Molekulargewicht von 1.000 bis 150.000 g/mol auf, mehr bevorzugt von 2.000 bis 90.000 g/mol auf. Im Rahmen dieser Anmeldung wird das gewichtsmittlere Molekulargewicht bevorzugt mittels Gel-Permeationschromatographie bestimmt.

Es ist bevorzugt, dass die als Hydrophilierungsmittel verwendeten Polymere im Wesentlichen keine Emulgatorwirkung zeigen. Das heißt, das verwendete Hydrophilierungsmittel sollte bevorzugt keine Kombination aus hydrophilen und hydrophoben Gruppen (insbesondere hydrophobe Fettsäuregruppen) aufweisen. Ferner ist es bevorzugt, dass das erfindungsgemäße Intermediat keine Polymere enthält, die ein gewichtsmittleres Molekulargewicht von mehr als 150.000 g/mol aufweisen. Derartige Polymere beeinflussen in der Regel die Auflöseeigenschaften in unerwünschter Weise.

Wird das als Hydrophilierungsmittel verwendete Polymer in Wasser in einer Menge von 2 Gew.-% gelöst, so zeigt die resultierende Lösung bevorzugt eine Viskosität von 0,1 bis 8 mPa/s, mehr bevorzugt von 0,5 bis 7 mPa/s, insbesondere von 1 bis 6 mPa/s, gemessen bei 25 °C und gemäß Ph. Eur., 6. Auflage, Kapitel 2.2.10 bestimmt.

Ferner umfasst das Hydrophilierungsmittel (ii) auch feste, nicht-polymere Verbindungen, die bevorzugt polare Seitengruppen aufweisen.

Das erfindungsgemäße Intermediat kann beispielsweise folgende hydrophile Polymere als Hydrophilierungsmittel umfassen: Polysaccharide, wie Hydroxypropylmethylcellulose (HPMC), Carboxymethylcellulose (CMC, insbesondere Natrium- und Calciumsalze), Ethylcellulose, Methylcellulose, Hydroxyethylcellulose, Ethylhydroxyethylcellulose, Hydroxypropylcellulose (HPC); Polyvinylpyrrolidon, Polyvinylalkohol, Polymere der Acrylsäure und deren Salze, Polyacrylamid, Polymethacrylate, Vinylpyrrolidon-Vinylacetat-Copolymere (beispielsweise Kollidon VA64, BASF), Polyalkylenglykole, wie Polypropylenglykol oder bevorzugt Polyethylenglykol, Co-blockpolymere des Polyethylenglykols, insbesondere Co-blockpolymere aus Polyethylenglykol und Polypropylenglykol (Pluronic®. BASF) sowie Gemische aus den genannten Polymeren.

Als Hydrophilierungsmittel (ii) besonders bevorzugt verwendet werden Polyvinylpyrrolidon, bevorzugt mit einem gewichtsmittleren Molekulargewicht von 10.000 bis 60.000 g/mol, insbesondere 12.000 bis 40.000 g/mol, Copolymer aus Vinylpyrrolidon und Vinylacetat, insbesondere mit einem gewichtsmittleren Molekulargewicht von 40.000 bis 70.000 g/mol und/oder Polyethylenglykol, insbesondere mit einem gewichtsmittleren Molekulargewicht von 2.000 bis 10.000 g/mol.

Als Hydrophilierungsmittel (ii) besonders bevorzugt verwendet werden Co-blockpolymere aus Polyethylenglykol und Polypropylenglykol, d.h. Polyoxyethylen Polyoxypropylen-Blockpolymerisate. Bevorzugt weisen diese ein gewichtsmittleres Molekulargewicht von 1.000 bis 20.000 g/mol, mehr bevorzugt von 1.500 bis 12.500 g/mol, insbesondere 5.000 bis 10.000 g/mol auf. Diese Blockpolymerisate sind bevorzugt erhältlich durch Kondensation von Propylenoxid mit Propylenglykol und nachfolgender Kondensation des entstandenen Polymers mit Ethylenoxid. Das heißt, bevorzugt liegt der Ethylenoxidanteil als "Endblock" vor. Bevorzugt weisen die Blockpolymerisate ein Gewichtsverhältnis von Propylenoxid zu Ethylenoxid von 50 : 50 bis 95 : 5, mehr bevorzugt von 70 : 30 bis 90 : 10 auf. Die Blockpolymerisate weisen bevorzugt eine Viskosität bei 25 °C von 200 bis 2000 mPas, mehr bevorzugt von 500 bis 1500 mPas, insbesondere von 800 bis 1200 mPas auf.

Im Rahmen dieser Erfindung können auch Gemische der vorstehend genannten Hydrophilierungsmittel eingesetzt werden. In einer möglichen Ausführungsform wird beispielsweise ein Gemisch aus Polyvinylpyrrolidon und Polyoxyethylen Polyoxypropylen-Blockpolymerisaten eingesetzt.

In einer bevorzugten Ausführungsform enthält das erfindungsgemäße Intermediat Cinacalcet oder ein pharmazeutisch verträgliches Salz davon, bevorzugt in nicht mikronisierter Form, und Hydrophilierungsmittel, wobei das

Gewichtsverhältnis von Wirkstoff (i) zu Hydrophilierungsmittel (ii) 5 : 1 bis 1 : 5, mehr bevorzugt 3 : 1 bis 1 : 3, noch mehr bevorzugt 2 : 1 bis 1 : 2, insbesondere ungefähr 1 : 1 beträgt.

Es ist bevorzugt, dass Art und Menge des Hydrophilierungsmittels so gewählt werden, dass mindestens 50 % der Oberfläche der resultierenden Intermediatteilchen mit Hydrophilierungsmittel bedeckt sind, mehr bevorzugt mindestens 60 % der Oberfläche, besonders bevorzugt mindestens 80 % der Oberfläche, insbesondere mindestens 95 % der Oberfläche.

Im Rahmen dieser Erfindung ist es besonders bevorzugt, dass Cinacalcet (i) und Hydrophilierungsmittel (ii) gemeinsam kompaktiert werden.

Die Kompaktierung kann in üblichen Kompaktiervorrichtungen erfolgen. Bevorzugt wird die Kompaktierung in einem Walzenkompaktierer durchgeführt. Die Walzkraft beträgt üblicherweise 5 bis 70 kN/cm, bevorzugt 10 bis 60 kN/cm, mehr bevorzugt 15 bis 50 kN/cm, insbesondere 15 bis 25 kN/cm.

Die Kompaktierungsbedingungen werden üblicherweise so gewählt, dass das erfindungsgemäße Intermediat in Form eines Kompaktats (Schülpe) vorliegt, wobei die Dichte des Intermediats 0,8 bis 1,3 $g/cm^3$, bevorzugt 0,9 bis 1,20 $g/cm^3$, insbesondere 1,01 bis 1,15 $g/cm^3$ beträgt.

Der Ausdruck "Dichte" bezieht sich hierbei bevorzugt auf die "Reindichte" (d.h. nicht auf die Schüttdichte oder Stampfdichte). Die Reindichte kann mit einem Gaspyknometer bestimmt werden. Vorzugsweise handelt es sich bei dem Gaspyknometer um ein Helium-Pyknometer, insbesondere wird das Gerät AccuPyc 1340 Helium Pyknometer des Herstellers Micromeritics, Deutschland verwendet.

Das erfindungsgemäße Intermediat findet zur Herstellung einer Tablette, bevorzugt zu einer Tablette mit sofortiger Freisetzung (im Englischen als "Immediate-Release Tablet" bekannt) Verwendung.

Gegenstand der Erfindung ist daher eine Tablette mit sofortiger Freisetzung, enthaltend

($\alpha$) erfindungsgemäßes Intermediat und
($\beta$) pharmazeutische Hilfsstoffe.

Hierbei handelt es sich um die dem Fachmann bekannten Hilfsstoffe ($\beta$), insbesondere solche, die im Europäischen Arzneibuch beschrieben sind.
Beispiele für verwendete Hilfsstoffe ($\beta$) sind Sprengmittel, Trennmittel, Füllstoffe. Zusätze zur Verbesserung der Pulverfließfähigkeit, Gleitmittel, Netzmittel, Gelbildner und/oder Schmiermittel.

Das Verhältnis Wirkstoff zu Hilfsstoffe wird bevorzugt so gewählt, dass die resultierenden Formulierungen

5 bis 60 Gew.-%. mehr bevorzugt 20 bis 45 Gew.-%, nicht-mikronisiertes, kristallines Cinacalcet und
40 bis 95 Ges.-%. mehr bevorzugt 55 bis 80 Gew.-%, pharmazeutisch verträgliche Hilfsstoffe enthalten.

Bei diesen Angaben wird die Menge an Hydrophilierungsmittel. die zur Herstellung des erfindungsgemäßen Intermediats verwendet wurde, als Hilfsstoff gerechnet. Das heißt, die Menge an Wirkstoff bezieht sich auf die Menge an nicht-mikronisiertem Cinacalcet, die in der fertigen Formulierung enthalten ist.

In einer bevorzugten Ausführungsform enthält die erfindungsgemäße Tablette 1 bis 40 Gew.-%, 5 bis 35 Gew.-%. mehr bevorzugt 10 bis 30 Gew.-%. besonders bevorzugt 15 bis 25 Gew.-% Sprengmittel, bezogen auf das Gesamtgewicht der Formulierung. Als Sprengmittel werden im allgemeinen Stoffe bezeichnet, die den Zerfall einer Darreichungsform, insbesondere einer Tablette, nach Einbringen in Wasser, beschleunigen. Geeignete Sprengmittel sind z.B. organische Sprengmittel wie Carrageenan, Croscarmellose, Natriumcarboxymethylstärke, Soja-polysaccharide und Crospovidon. Alternativ können alkalische Sprengmittel verwendet werden. Unter alkalischen Sprengmitteln sind Sprengmittel zu verstehen, die beim Lösen in Wasser einen pH-Wert von mehr als 7,0 erzeugen.

Besonders bevorzugt wird Crospovidon und/oder Croscarmellose als Sprengmittel, insbesondere in den oben genannten Mengen, verwendet.

In einer bevorzugten Ausführungsform enthält die erfindungsgemäße Tablette einen (oder mehrere) pH-Anpasser. Im Allgemeinen sind Säuren oder Puffersubstanzen als pH-Anpasser geeignet.

Als Säuren werden üblicherweise organische Säuren mit einem pKs-Wert von 2 bis 6, bevorzugt von 3 bis 5, verwendet, die eine Wasserlöslichkeit von > 1 g/250 ml bei 20°C. vorzugsweise von > 1g/160 ml bei 25°C aufweisen. Beispiele sind Fumarsäure, Weinsäure, Zitronensäure, Äpfelsäure, Glutaminsäure, Asparaginsäure und Gemische daraus.

Unter Puffersubstanzen versteht man Substanzgemische, deren pH-Wert in wässrigen Lösungen im Wesentlichen unempfindlich gegen geringe Mengen an Säure- oder Basezusatz sind. Hierfür eignen sich bevorzugt äquimolekulare Mischungen schwacher Säuren und ihre Alkalisalze. Analoges gilt für die Basen. Geeignete Puffersubstanzen sind beispielsweise ein Acetatpuffer, Citrat- oder ein Phosphatpuffer. Bei dem Acetatpuffer handelt es sich bevorzugt um ein Gemisch aus $CH_3COOH$ und $CH_3COOM$. Bei dem Phosphatpuffer handelt es sich bevorzugt um ein Gemisch aus $H_2PO_4M$ und $HPO_4M_2$. M ist ein Alkalimetall, bevorzugt Natrium. Der Acetatpuffer ist besonders bevorzugt.

Die pH-Anpasser werden üblicherweise so gewählt, dass ein pH-Wert von 3,5 bis 5,5, bevorzugt von 4 bis 5, eingestellt werden kann. Der Acetatpuffer ist als pH-Anpasser besonders bevorzugt.

Die erfindungsgemäße Tablette enthält in dieser Ausführungsform üblicherweise 0,1 bis 15 Ges.-% pH-Anpasser, bevorzugt 0,5 bis 10 Gew.-%, mehr bevorzugt 1 bis 8 Gew-%, bezogen auf das Gesamtgewicht der Tablette.

Die Verwendung eines pH-Anpassers trägt insbesondere dazu bei, dass die erfindungsgemäße Tablette unabhängig von den Mahlzeiten verabreicht werden kann. Ferner führt die Verwendung des pH-Anpassers zur Verbesserung der Löslichkeit. Die Erfinder der vorliegenden Anmeldung haben ferner gefunden, dass dieser Effekt auch unabhängig von einer Kompaktierung eintreten kann.

Gegenstand der Erfindung ist daher eine orale Darreichungsform enthaltend Cinacalcet und einen pH-Anpasser. Der pH-Anpasser ist bevorzugt dazu geeignet, einen pH-Wert von 3,5 bis 5,5, bevorzugt von 4 bis 5, einzustellen. Die Verabreichung erfolgt insbesondere unabhängig von den Mahlzeiten. Unter dem Begriff "Verabreichung unabhängig von den Mahlzeiten" ist zu verstehen, dass der Patient das Arzneimittel zu den Mahlzeiten einnehmen kann, aber nicht zwingend zu den Mahlzeiten einnehmen muss.

Ebenfalls ist Gegenstand der Erfindung die Verwendung eines pH-Anpassers, der dazu geeignet ist, einen pH-Wert von 3,5 bis 5,5, bevorzugt von 4 bis 5, einzustellen, zur Herstellung einer pharmazeutischen Formulierung, die Cinacalcet als Wirkstoff enthält.

Bei der oralen Darreichungsform handelt es sich z.B. um Kapseln, Pulver oder Granulat zur Abfüllung in Sachets oder Tabletten. Tabletten sind bevorzugt.

Für diese erfindungsgemäßen oralen Darreichungsformen und die erfindungsgemäße Verwendung finden die vorstehenden Ausführungen zu den pH-Anpassern Anwendung.

Die erfindungsgemäße Tablette enthält bevorzugt Füllstoffe. Unter Füllstoffe sind im Allgemeinen Stoffe zu verstehen, die zur Bildung des Tablettenkörpers bei Tabletten mit geringen Wirkstoffmengen (z.B. kleiner 70 Gew.-%) dienen. Das heißt, Füllstoffe erzeugen durch "Strecken" der Wirkstoffe eine ausreichende Tablettiermassse. Füllstoffe dienen üblicherweise also dazu, eine geeignete Tablettengröße zu erhalten.

Beispiele für bevorzugte Füllstoffe sind Lactose, Lactosederivate, Stärke, Stärkederivate, behandelte Stärke, Chitin, Cellulose und Derivate davon, Calciumphosphat, Saccharose, Calciumcarbonat, Magnesiumcarbonat, Magnesiumoxid, Maltodextrin, Calciumsulfat, Dextrate, Dextrin, Dextrose, hydrogeniertes Pflanzenöl, Kaolin, Natriumchlorid, und/oder Kaliumchlorid. Ebenfalls kann Prosolv® (mit $SiO_2$ modifizierte mikrokristalline Cellulose, Rettenmater & Söhne, Deutschland) verwendet werden.

Ebenfalls können als Füllstoffe Zuckeralkohole und/oder Disaccharide wie Mannitol, Sorbitol, Xylitol, Isomalt, Glucose, Fructose, Maltose und Gemische daraus verwendet werden. Der Begriff Zuckeralkohole umfasst hier auch Monosaccharide.

Füllstoffe werden üblicherweise in einer Menge von 1 bis 80 Gew.-%, mehr bevorzugt von 30 bis 60 Gew.-%. insbesondere von 20 bis 40 Gew.-%. bezogen auf das Gesamtgewicht der Formulierung verwendet.

Die erfindungsgemäße Tablette kann ferner Zusätze zur Verbesserung der Pulverfließfähigkeit enthalten. Ein Beispiel für einen Zusatz zur Verbesserung der Pulverfließfähigkeit ist disperses Siliciumdioxid, z.B. bekannt unter dem Handelsnamen Aerosil®. Bevorzugt wird Siliciumdioxid mit einer spezifischen Oberfläche von 50 bis 400 $m^2/g$, bestimmt nach Gasadsorption gemäß Ph. Eur., 6. Auflage 2.9.26., verwendet.

Zusätze zur Verbesserung der Pulverfließfähigkeit werden üblicherweise in einer Menge von 0,1 bis 5 Gew.-%. z.B. 1,5 bis 4 Gew.-%, bezogen auf das Gesamtgewicht der Formulierung verwendet.

Ferner können Schmiermittel verwendet werden. Schmiermittel dienen im Allgemeinen zur Verringerung der Gleitreibung. Insbesondere soll die Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat (Pruv®) und/oder Magnesiumstearat dar.

Schmiermittel werden üblicherweise in einer Menge von 0,1 bis 5 Gew.-%, bevorzugt 1,0 bis 3 Gew.-%. bezogen auf das Gesamtgewicht der Formulierung, verwendet.

Weiterhin können Trennmittel verwendet werden. Unter Trennmitteln werden üblicherweise Stoffe verstanden, welche die Agglomeration im Kernbett vermindern. Beispiele sind Talkum, Silicagel, Polyethylenglykol (bevorzugt mit 2.000 bis 10.000 g/mol gewichtsmittlerem Molekulargewicht) und/oder Glycerolmonostearat.

Es liegt in der Natur von pharmazeutischen Hilfsstoffen, dass diese teilweise mehrere Funktionen in einer pharmazeutischen Formulierung wahrnehmen. Im Rahmen dieser Erfindung gilt zur unzweideutigen Abgrenzung daher bevorzugt die Fiktion, dass ein Stoff, der als ein bestimmter Hilfsstoff verwendet wird, nicht zeitgleich auch als weiterer pharmazeutischer Hilfsstoff eingesetzt wird. Beispielsweise wird Sorbitol - sofern als Füllstoff eingesetzt - nicht auch zusätzlich als Hydrophilierungsmittel eingesetzt. Ebenfalls wird beispielsweise mikrokristalline Cellulose - sofern als Hydrophilierungsmittel eingesetzt - nicht auch zusätzlich als Sprengmittel eingesetzt (obwohl mikrokristalline Cellulose auch eine gewisse Sprengwirkung zeigt).

In einer bevorzugten Ausführungsform enthält die erfindungsgemäße Tablette (bezogen auf das Gesamtgewicht des Tablettenkerns) folgende Bestandteile:

15 bis 40 Gew.-% Cinacalcet
15 bis 35 Gew.-% Hydrophilierungsmittel
15 bis 40 Gew.-% Füllstoff
15 bis 35 Gew.-% Sprengmittel und
1 bis 4 Gew.-% Schmiermittel.

In einer alternativen bevorzugten Ausführungsform enthält die erfindungsgemäße Tablette (bezogen auf das Gesamtgewicht des Tablettenkerns) folgende Bestandteile: mehr als 40 bis 60 Gew.-% Cinacalcet

15 bis 35 Gew.-% Hydrophilierungsmittel
0 bis 10 Gew.-% Füllstoff
15 bis 35 Gew.-% Sprengmittel und
1 bis 4 Gew.-% Schmiermittel.

Die erfindungsgemäßen Tabletten enthalten bevorzugt keine Polymere, welche zu einer verzögerten Freisetzung führen. Insbesondere ist es bevorzugt, dass die erfindungsgemäßen Tabletten keine Polymere enthalten, die ein Molekulargewicht von mehr als 150.000 g/mol aufweisen.

Gegenstand der Erfindung ist ferner ein Verfahren zur Herstellung der erfindungsgemäßen Tabletten, umfassend die Schritte

(a) Vermischen von (i) kristallinem Cinacalcet oder dessen pharmazeutisch verträglichen Salzen, mit (ii) einem Hydrophilierungsmittel sowie gegebenenfalls weiteren pharmazeutischen Hilfsstoffen;
(b) Kompaktierung zu einer Schülpe;
(c) Granulierung der Schülpe;
(d) Kompression der resultierenden Granulate zu Tabletten, gegebenenfalls unter Zusatz weiterer pharmazeutischer Hilfsstoffe; und
(e) gegebenenfalls Befilmung der Tabletten.

Grundsätzlich finden alle Erläuterungen, die vorstehend zu bevorzugten Ausführungsformen des erfindungsgemäßen Intermediats ausgeführt wurden, auch auf das erfindungsgemäße Verfahren Anwendung.

In einer bevorzugten Ausführungsform werden in Schritt (a) des erfindungsgemäßen Verfahrens (i) kristallines Cinacalcet oder dessen pharmazeutisch verträglichen Salze mit (ii) einem Hydrophillerungsmittel sowie gegebenenfalls weiteren - vorstehend beschriebenen - pharmazeutischen Hilfsstoffen (β) vermischt.

Wie vorstehend erwähnt, umfasst das Hydrophilierungsmittel bevorzugt kein Polymer mit einem gewichtsmittleren Molekulargewicht von mehr als 150.000 g/mol. Gleiches gilt für die pharmazeutischen Hilfsstoffe, die im Schritt (a) (und/oder auch im Schritt (d)) des erfindungsgemäßen Verfahrens zugegeben werden.

Die Vermischung kann in üblichen Mischern erfolgen. Alternativ kann die Vermischung von Wirk- und Hilfsstoffen auch nach dem Granulierschritt (c) erfolgen.

Alternativ ist es ebenso möglich, dass das erfindungsgemäße Intermediat mit einem Teil der Hilfsstoffe (z.B. 50 bis 95 %) vor der Kompaktierung (b) vermischt wird, und dass der verbleibende Teil der Hilfsstoffe nach dem Granulierschritt (c) zugegeben wird. Im Falle der Mehrfachkompaktierung sollte das Zumischen der Hilfsstoffe bevorzugt vor dem ersten Kompaktierschritt, zwischen mehreren Kompaktierschritten oder nach dem letzten Granulierschritt erfolgen.

In einer bevorzugten Ausführungsform werden in Schritt (a)

100 % des eingesetzten Cinacalcet
100 % des eingesetzten Hydrophilierungsmittels
gegebenenfalls 20 bis 70 % des eingesetzten Füllstoffs und
gegebenenfalls 30 bis 70 % des eingesetzten Sprengmittels und
gegebenenfalls 10 bis 40 % des eingesetzten Schmiermittels

vermischt. Die verbleibenden optionalen Mengen an Füllstoff. Sprengmittel und Schmiermittel werden anschließend in Schritt (d) zugegeben.

Im Schritt (b) des erfindungsgemäßen Verfahrens wird das Gemisch aus Schritt (1) zum erfindungsgemäßen Intermediat kompaktiert. Hierbei ist bevorzugt, dass es sich um eine Trockenkompaktierung handelt (d.h. die Kompaktierung erfolgt bevorzugt in Abwesenheit von Lösungsmitteln, insbesondere in Abwesenheit von organischen Lösungsmitteln).

Die Kompaktierungsbedingungen im Schritt (b) werden bevorzugt so gewählt, dass die Schülpe eine Dichte von 0,8 bis 1,3 $g/cm^3$, bevorzugt 0,9 bis 1,20 $g/cm^3$, insbesondere 1,01 bis 1,15 $g/cm^3$ aufweist.

Der Ausdruck "Dichte" bezieht sich hierbei bevorzugt auf die "Reindichte" und wird wie vorstehend beschrieben bestimmt.

Die Kompaktierung wird bevorzugt in einem Walzengranulator durchgeführt.

Üblicherweise beträgt die Walzkraft 5 bis 70 kN/cm, bevorzugt 10 bis 60 kN/cm, mehr bevorzugt 15 bis 50 kN/cm, insbesondere 15 bis 25 kN/cm.

Die Spaltbreite des Walzengranulators beträgt beispielsweise 0,8 bis 5 mm, bevorzugt 1 bis 4 mm, mehr bevorzugt 1,5 bis 3 mm, insbesondere 1,8 bis 2,8 mm.

Die verwendete Kompaktiervorrichtung weist bevorzugt eine Kühlvorrichtung auf. Insbesondere wird derart gekühlt, dass die Temperatur des Kompaktats 55 °C nicht überschreitet.

In Schritt (c) des erfindungsgemäßen Verfahrens wird die Schülpe granuliert. Die Granulierung kann mit im Stand der Technik bekannten Verfahren erfolgen.

In einer bevorzugten Ausführungsform werden die Granulierungsbedingungen so gewählt, dass die resultierenden Teilchen (Granulate) eine volumenmittlere Teilchengröße (D50-Wert) von 75 bis 600 µm aufweisen, mehr bevorzugt von 120 bis 500 µm. noch mehr bevorzugt 150 bis 400 µm, insbesondere von 200 bis 350 µm. Die volumenmittlere Teilchengröße wird (wie vorstehend beschrieben) mittels Laserdiffraktometrie bestimmt (unter Verwendung eines Mastersizer 2000 von Malvern Instruments). In einer alternativen Ausführungsform werden die Granulierungsbedingungen so gewählt, dass die resultierenden Teilchen (Granulate) eine gewichtsmittlere Teilchengröße (D50-Wert) von 75 bis 600 µm aufweisen, mehr bevorzugt von 120 bis 500 µm, noch mehr bevorzugt von 150 bis 400 µm, insbesondere von 200 bis 350 µm. Die gewichtsmittlere Teilchengröße wird mittels Siebanalyse bestimmt (unter Verwendung einer Retsch® AS 2000, Amplitude 1,5 sec., Intervall 10 min.. Probeneinwaage 15,8 g).

In einer bevorzugten Ausführungsform erfolgt die Granulierung in einer Siebmühle. In diesem Fall beträgt die Maschenweite des Siebeinsatzes üblicherweise 0,1 bis 5 mm, bevorzugt 0,5 bis 3 mm, mehr bevorzugt 0,75 bis 2 mm, insbesondere 0,8 bis 1,8 mm.

Beispielsweise wird zur Granulierung eine Comil® U5 (Quadro Engineering, USA) verwendet.

Weiterhin werden die Granulierbedingungen bevorzugt so gewählt, dass die resultierenden Granulate eine Schüttdichte von 0,3 bis 0,85 g/ml, mehr bevorzugt 0,4 bis 0,8 g/ml, insbesondere 0,5 bis 0,7 g/ml aufweisen. Der Hausner-Faktor liegt üblicherweise im Bereich von 1,02 bis 1,3, mehr bevorzugt von 1,03 bis 1,25 und insbesondere von 1,04 bis 1.15. Unter "Hausner-Faktor" wird hierbei das Verhältnis von Stampfdichte zu Schüttdichte verstanden. Die Bestimmung von Schütt- und Stampfdichte erfolgt gemäß USP 24, Test 616 "Bulk Density and Tapped Density".

Gegebenenfalls können die erfindungsgemäßen Intermediate eine nicht ausreichend rauhe Oberfläche aufweisen, so dass der vorstehend beschriebene Kompaktierungsschritt (ii) erschwert wird. Daher kann, je nach Oberflächenbeschaffenheit, der Kompaktierschritt (b) und der Granulierschritt (c) bei Bedarf wiederholt werden.

In einer bevorzugten Ausführungsform wird daher das erfindungsgemäße Verfahren derart angepasst, dass eine Mehrfachkompaktierung erfolgt, wobei das aus Schritt (c) resultierende Granulat einmal oder mehrmals zur Kompaktierung (b) rückgeführt wird.

Bevorzugt wird das Granulat aus Schritt (c) 1 bis 5 mal rückgeführt. insbesondere 2 bis 3 mal.

In Schritt (d) des erfindungsgemäßen Verfahrens werden die in Schritt (c) erhaltenen Granulate zu Tabletten verpresst. d.h. es erfolgt eine Kompression zu Tabletten. Die Kompression kann mit im Stand der Technik bekannten Tablettiermaschinen wie Exzenterpressen oder Rundlaufpressen erfolgen. Im Falle von Rundlaufpressen wird üblicherweise eine Presskraft von 2 bis 40 kN, bevorzugt von 2,5 bis 35 kN, angewandt. Beispielsweise wird die Presse Fette® 102i (Fette GmbH. Deutschland) verwendet. Im Falle von Exzenterpressen wird üblicherweise eine Presskraft von 1 bis 20 kN, bevorzugt von 2,5 bis 10 kN, angewandt. Beispielsweise wird die Korsch® EK0 verwendet.

Verfahrensschritt (d) erfolgt bevorzugt in Abwesenheit von Lösungsmitteln, insbesondere organischen Lösungsmitteln, d.h. als Trockenkompression.

In Schritt (d) des erfindungsgemäßen Verfahrens können den Granulaten aus Schritt (c) pharmazeutische Hilfsstoffe (β) zugegeben werden. Hierbei wird auf die vorstehend ausgeführten Erläuterungen zu geeigneten Hilfsstoffen verwiesen.

Gegenstand der Erfindung ist nicht nur das erfindungsgemäße Verfahren, sondern auch die mit diesem Verfahren hergestellten Tabletten. Es wurde gefunden, dass die mit diesem Verfahren hergestellten Tabletten bevorzugt eine bimodale Porengrößenverteilung aufweisen.

Diese erfindungsgemäße Tablette entsteht, wenn das Granulat aus Verfahrensschritt (c) komprimiert wird. Dieses Komprimat besteht aus Feststoff und Poren. Die Porenstruktur kann durch Bestimmung der Porengrößenverteilung näher charakterisiert werden.

Die Porengrößenverteilung wurde mittels Quecksilberporosimetrie bestimmt. Quecksilberporosimetriemessungen erfolgten mit dem Porosimeter "Poresizer" der Firma Micromeritics, Norcross, USA. Die Porengrößen wurden hierbei

unter Annahme einer Oberflächenspannung von Quecksilber von 485 mN/m berechnet. Aus dem kumulativen Porenvolumen wurde die Porengrößenverteilung als Summenverteilung beziehungsweise Anteil der Porenfraktionen in Prozent errechnet. Der durchschnittliche Porendurchmesser (4V/A) wurde aus dem gesamten spezifischen Quecksilberintrusionsvolumen ($Vges_{int}$) und der gesamten Porenfläche ($Agesp_{por}$) nach folgender Gleichung ermittelt.

$$4V/A = \frac{4 \cdot Vges_{int}\,[ml/g]}{Ages_{por}\,[m^2/g]}$$

Unter "bimodaler Porengrößenverteilung" wird verstanden, dass die Porengrößenverteilung zwei Maxima aufweist.

Bevorzugt liegt ein Maximum der Porengrößenverteilung bei einer Porengröße von 0,01 bis 0,4 µm, mehr bevorzugt bei 0,05 bis 0,3 µm, insbesondere bei 0,1 bis 0.2 µm. Ein zweites Maximum liegt bevorzugt bei einer Porengröße von 0,4 bis 5 µm, mehr bevorzugt 0,5 bis 2 µm, insbesondere 0,6 bis 1.2 µm.

Insbesondere ist eine Tablette bevorzugt, die einerseits die vorstehend erläuterte Porengrößenverteilung aufweist und andererseits die vorstehend erläuterten pH-Anpasser enthält. Diese Tabletten sind insbesondere zur Verabreichung unabhängig von den Mahlzeiten geeignet.

Bei den durch das erfindungsgemäße Verfahren hergestellten Tabletten kann es sich um Tabletten, die unzerkaut geschluckt werden (unbefilmt oder bevorzugt befilmt), handeln. Ebenfalls kann es sich um Kautabletten oder um Disperstabletten handeln. Unter "Disperstablette" wird hierbei eine Tablette zur Herstellung einer wässrigen Suspension zum Einnehmen verstanden.

Im Falle von Tabletten, die unzerkaut geschluckt werden, ist es bevorzugt, dass diese mit einer Filmschicht im Schritt (e) des erfindungsgemäßen Verfahrens überzogen werden. Die vorstehend genannten Verhältnisse von Wirkstoff zu Hilfsstoff beziehen sich jedoch auf die unlackierte Tablette.

Für die Befilmung werden bevorzugt makromolekulare Stoffe verwendet, beispielsweise modifizierte Cellulosen, Polymethacrylate, Polyvinylpyrrolidon. Polyvinylacetatphthalat. Zein und/oder Schellack.

Bevorzugt verwendet wird HPMC, insbesondere HPMC mit einem gewichtsmittleren Molekulargewicht von 10.000 bis 150.000 g/mol und/oder einem durchschnittlichen Substitutionsgrad an $-OCH_3$-Gruppen von 1,2 bis 2,0.

Die Schichtdicke des Überzugs beträgt bevorzugt 2 bis 100 µm.

Die Tablettierbedingungen werden bevorzugt so gewählt. dass die resultierenden Tabletten ein Verhältnis von Tablettenhöhe zu Gewicht von 0.005 bis 0.3 mm/mg. besonders bevorzugt 0.05 bis 0,2 mm/mg aufweisen.

Ferner weisen die resultierenden Tabletten bevorzugt eine Härte von 70 bis 200 N. besonders bevorzugt von 100 bis 150 N auf. insbesondere wenn das Tablettengewicht mehr als 200 mg beträgt. Sofern das Tablettengewicht 200 mg oder weniger beträgt, weisen die resultierenden Tabletten bevorzugt eine Härte von 30 bis 100 N, besonders bevorzugt von 50 bis 70 N, auf. Die Härte wird gemäß Ph.Eur. 6.0, Abschnitt 2.9.8 bestimmt.

Zudem zeigen die resultierenden Tabletten bevorzugt eine Friabilität von kleiner 3 %, besonders bevorzugt von kleiner 1 %, insbesondere von kleiner 0,8 auf. Die Friabilität wird gemäß Ph.Eur. 6.0, Abschnitt 2.9.7 bestimmt.

Schließlich weisen die erfindungsgemäßen Tabletten üblicherweise eine "Content Uniformity" von 95 bis 105 % vom durchschnittlichen Gehalt, bevorzugt von 98 bis 102 %, insbesondere von 99 bis 101 % vom durchschnittlichen Gehalt auf. Die "Content Uniformity" wird gemäß Ph. Eur.6.0, Abschnitt 2.9.6. bestimmt.

Das Freisetzungsprofil der erfindungsgemäßen Tabletten weist gemäß USP-Methode (Paddle, 900 ml 0,1 N HCl, pH 1,2, 37 °C. 75 rpm) nach 10 Minuten üblicherweise einen freigesetzten Gehalt von mindestens 30 %, bevorzugt mindestens 50 %, insbesondere mindestens 70 % auf.

Die vorstehenden Angaben zu Härte, Friabilität, Content Uniformity und Freisetzungsprofil beziehen sich hierbei bevorzugt auf die unbefilmte Tablette.

Alternativ zur Verpressung in Tabletten können die in Schritt (c) des erfindungsgemäßen Verfahrens resultierenden Granulate auch - gegebenenfalls unter Zusetzung weiterer pharmazeutischer Hilfsstoffe - zu einer partikulären Darreichungsform verarbeitet werden, beispielsweise durch Abfüllung in Kapseln oder in Sachets.

In einer bevorzugten Ausführungsform wird hierfür Sprengmittel in einer Menge von 10 bis 30 Gew.-% verwendet, bezogen auf das Gesamtgewicht der oralen Darreichungsform. In einer weiteren bevorzugten Ausführungsform wird hierfür ein Polyoxyethylen Polyoxypropylen-Blockpolymerisat als Hydrophilierungsmittel verwendet, insbesondere wie vorstehend näher beschrieben. In einer weiteren bevorzugen Ausführungsform beträgt der Gehalt an Cinacalcet 20 bis 60 Gew.-%, insbesondere 40 bis 60 Gew.-%.

Die Erfindung soll anhand der nachfolgenden Beispiele veranschaulicht werden.

## BEISPIELE

### Beispiel 1

Folgende Formulierung wurde eingesetzt:
Kern:

| | |
|---|---|
| Cinacalcet-Hydrochlorid (D50 101 μm, Wassergehalt 0,06 %) | 33,06 mg |
| Polyvinylpyrrolidon (Mw 30.000) | 10 mg |
| PEG 6000 | 15 mg |
| Prosolv® (Füllstoff) | 50 mg |
| Weinsäure | 60 mg |
| Magnesiumstearat | 1,8 mg |
| Crospovidon | 4 mg |

Film:

| | |
|---|---|
| HPMC (Mw 100.000) | 1,5 mg |
| Titandioxid | 0,4 mg |
| Farbstoff | 0,1 mg |

Cinacalcet-Hydrochlorid wurde mit Polyvinylpyrrolidon und PEG 6000 zusammen mit 70 Gew.-% des Prosolvs® und Crospovidons zu einer Schülpe (= Intermediat) auf einen Walzenkompaktor kompaktiert. Anschließend wurde die Schülpe mit einem Sieb (Comil U5, 1,0 mm) aufgesiebt. Anschließend wurde das resultierende Granulat mit den restlichen Kern-Hilfsstoffen gemischt (Turbula 10B) und zu Tabletten verpresst (Fette 102i). Anschließend erfolgte die Befilmung des Tablettenkerns.

### Beispiel 2

Folgende Formulierung wurde eingesetzt:
Kern:

| | |
|---|---|
| Cinacalcet-Hydrochlorid (D50 101 μm), | 33,0 mg |
| Polyoxyethylen Polyoxypropylen-Blockpolymerisat (Mw ca. 8350) | 30,0 mg |
| Sorbitol (Füllstoff) | 38,0 mg |
| Natriumstearylfumarat | 4,00 mg |
| Crospovidon | 40,0 mg |

Film:

| | |
|---|---|
| Opadry® AMB | 6,40 mg |

Das Herstellverfahren umfasste folgende Schritte:

- Cinacalcet HCl, Polyoxyethylen Polyoxypropylen-Blockpolymerisat, 20 mg Crospovidon, 20 mg Sorbitol und 1,5 mg Natriumstearylfumarat wurden in einem Turbula® TBD 10 (Freifallmischer) 5 min lang vorgemischt,
- das Vorgemisch wurde gesiebt (Maschenweite 0,315 mm) und weitere 10 min gemischt,
- es wurde mittels einer Korsch® EKO (Exzenter Presse, 18 kN, 20 mm biplan) zusammengepresst und mittels Sieben granuliert (Maschenweite 0,6 mm),
- das Granulat wurde zusammen mit dem restlichen Crospovidon und Sorbitol 10 min lang gemischt,
- Natriumstearylfumarat wurde durch Sieben hinzugefügt (Maschenweite 0,3 mm) und weitere 5 min lang gemischt,
- die erhaltene Mischung wurde zu Tabletten verpresst (9,7 x 5 r 3,6; 4,5 kN; 50 N), und
- die Tabletten wurden mit einer Opadry® AMB-Lösung überzogen.

Die resultierenden Tabletten zeigten vorteilhafte Löslichkeitseigenschaften, die nach Lagerung über drei Monate (bei 40 °C, 75 % Luftfeuchte) erhalten blieben, siehe Beispiel 4.

**Beispiel 3**

Folgende Formulierung wurde eingesetzt:
Kern:

| | |
|---|---|
| Cinacalcet-Hydrochlorid (D50 101 µm), | 33,0 mg |
| Polyoxyethylen Polyoxypropylen-Blockpolymerisat (Mw ca. 8350) | 16,5 mg |
| Natriumstearylfumarat | 3,00 mg |
| Crospovidon | 17,0 mg |

Film:

| | |
|---|---|
| Opadry® AMB | 4.0 mg |

Das Herstellverfahren umfasste folgende Schritte:

- ■ Cinacalcet HCl, Polyoxyethylen Polyoxypropylen-Blockpolymerisat, 9 mg Crospovidon und 1 mg Natriumstearylfumarat wurden in einem Turbula®TBD 10 (Freifallmischer) 5 min. lang gemischt,
- ■ das Vorgemisch wurde gesiebt (Maschenweite 0,6 mm) und weitere 10 min. lang gemischt,
- ■ es wurde mittels einer Korsch® EKO (Exzenter Presse, 18 kN, 20 mm biplan) zusammengepresst und mittels Sieben (Maschenweite 0,6 mm) granuliert,
- ■ das Granulat wurde zusammen mit dem restlichen Crospovidon 10 min lang gemischt,
- ■ Natriumstearylfumarat wurde durch Sieben hinzugefügt (Maschenweite 0,3 mm) und weitere 5 min lang gemischt,
- ■ die erhaltene Mischung wurde zu Tabletten verpresst (6 mm r 7,5, 5 kN, 50 N), und
- ■ die Tabletten wurden mit einer Opadry® AMB-Lösung überzogen.

Die resultierenden Tabletten zeigten vorteilhafte Löslichkeitseigenschaften (> 80 % nach 15 min, > 95 % nach 30 min), die nach Lagerung über drei Monate (40 °C, 75 % Luftfeuchte) erhalten blieben.

**Vergleichsbeispiel 1**

Zum Vergleich wurden Tabletten gemäß WO 2005/34928 A1 (Absatz [0057]), enthaltend 30 mg mikronisiertes Cinacalcet-HCl, mittels Feuchtgranulation hergestellt. Das Löslichkeitsverhalten wurde in Beispiel 4 untersucht.

**Beispiel 4**

Das in-vitro Löslichkeitsverhalten von (unbefilmten) Tabletten gemäß Beispiel 2 und Vergleichsbeispiel 1 wurde gemäß USP (Paddle, 900 ml 0,1 N HCl, pH 1,2, 37 °C. 75 rpm) vor und nach Lagerung (40 °C, 75 % rel. Luftfeuchte) untersucht.

| Beispiel | Rührzeit | Menge ohne Lagerung | Menge nach 2 Wochen | Menge nach 4 Wochen | Menge nach 8 Wochen | Menge nach 12 Wochen |
|---|---|---|---|---|---|---|
| Beispiel 2 | 15 | 96,4 | 101,7 | 101,5 | 103,1 | 101,0 |
| Vergleichsbeispiel 1 | 15 | 83,1 | 86,5 | 84,3 | 83,9 | 83,7 |
| | | | | | | |

Die Messung zeigt, dass die erfindungsgemäßen Tabletten ein sehr gutes Löslichkeitsverhalten auch nach Lagerung aufweisen, wobei eine Mikronisierung des Wirkstoffs vermieden werden konnte.

**Beispiel 5**

Es wurde eine Gehaltsbestimmung sowie eine Messung der Verunreinigung mittels HPLC Methode von Tabletten gemäß Beispiel 2 vor und nach Lagerung (40 °C, 75 % rel. Luftfeuchte) durchgeführt.

HPLC-Parameter:

Säule: X-Bridge C18 150 x 4,6 mm, 3.5 μm,
Durchfluss: 0,9 ml/min.
Säulentemperatur: 60°C,
Injektionsvolumen: 2 μl,
Elutionsmittel A: 25 mmol/l $KH_2PO_4$* $H_2O$ pH 3,00 ± 0.05
Elutionsmittel B: Acetonitril

| Pumpengradient: | Zeit [min] | % B |
|---|---|---|
| | 0 | 25 |
| | 3 | 25 |
| | 22 | 65 |
| | 25 | 25 |

Wellenlänge: 225 nm.
Proben-Lösungsmittel: Wasser / Acetonitril 50/50
Probenkonzentrierung: 450 μg/ml

| | ohne Lagerung | nach 4 Wochen | nach 12 Wochen |
|---|---|---|---|
| Gehalt | 97,2 | 96,83 | 99,27 |
| Verunreinigung total | 0,03 | 0,06 | 0,08 |

Die Bestimmung zeigt, dass die erfindungsgemäßen Tabletten eine sehr gute Lagerstabilität aufweisen.

## Patentansprüche

1. Intermediat, erhältlich durch gemeinsames Kompaktieren von

   (i) kristallinem Cinacalcet oder eines pharmazeutisch verträglichen Salzes davon, mit
   (ii) einem Hydrophilierungsmittel.

2. Intermediat nach Anspruch 1, wobei die Kompaktierung in einem Walzenkompaktierer durchgeführt wird und die Walzkraft 5 bis 70 kN/cm, bevorzugt 10 bis 50 kN/cm beträgt.

3. Intermediat nach Anspruch 1 oder 2, wobei die Dichte des Intermediats 0,8 bis 1,3 g/$cm^3$, bevorzugt 0,9 bis 1,20 g/$cm^3$ beträgt.

4. Intermediat nach einem der Ansprüche 1 bis 3, wobei kristallines Cinacalcet oder ein pharmazeutisch verträgliches Salz davon mit einer spezifischen Oberfläche von 0,01 bis 8 $m^2$/g eingesetzt wird.

5. Intermediat nach einem der Ansprüche 1 bis 4, wobei als Hydrophilierungsmittel hydrophile Polymere mit einem gewichtsmittleren Molekulargewicht von weniger als 150.000 g/mol eingesetzt werden.

6. Intermediat nach Anspruch 5, wobei als Hydrophilierungsmittel Polyvinylpyrrolidon, ein Copolymer aus Vinylpyrrolidon und Vinylacetat und/oder Polyethylenglykol verwendet wird.

7. Intermediat nach einem der Ansprüche 1 bis 6, wobei das Gewichtsverhältnis von Komponente (i) zu Komponente

(ii) 1 : 5 bis 5 : 1 beträgt.

**8.** Tablette mit sofortiger Freisetzung, enthaltend
(α) ein Intermediat gemäß einem der Ansprüche 1 bis 7, und
(β) pharmazeutische Hilfsstoffe.

**9.** Tablette nach Anspruch 8, **dadurch gekennzeichnet, dass** die Komponente (β) Sprengmittel enthält.

**10.** Tablette nach Anspruch 9, wobei das Sprengmittel in einer Menge von 10 bis 30 Gew.-%, bezogen auf das Gesamtgewicht der Formulierung, vorliegt.

**11.** Tablette nach einem der Ansprüche 8 bis 10. wobei die Tablette einen Gehalt an Cinacalcet von 40 bis 60 Gew.-% aufweist.

**12.** Verfahren zur Herstellung einer Tablette gemäß einem der Ansprüche 8 bis 11. umfassend die Schritte

(a) Vermischen von (i) kristallinem Cinacalcet oder dessen pharmazeutisch verträglichen Salzen, mit (ü) einem Hydrophilierungsmittel sowie gegebenenfalls weiteren pharmazeutischen Hilfsstoffen;
(b) Kompaktierung zu einer Schülpe;
(c) Granulierung der Schülpe;
(d) Kompression der resultierenden Granulate zu Tabletten, gegebenenfalls unter Zusatz weiterer pharmazeutischer Hilfsstoffe; und
(e) gegebenenfalls Befilmung der Tabletten.

**13.** Verwendung eines pH-Anpassers, der dazu geeignet ist, einen pH-Wert von 3,5 bis 5,5, bevorzugt von 4 bis 5, einzustellen, zur Herstellung einer pharmazeutischen Formulierung, die Cinacalcet als Wirkstoff enthält.

## Claims

**1.** An intermediate, obtainable by jointly compacting

(i) crystalline cinacalcet or a pharmaceutically acceptable salt thereof, with
(ii) a hydrophilising agent.

**2.** The intermediate as claimed in claim 1, wherein the compacting is performed in a roller compacter and the rolling force is 5 to 70 kN/cm, preferably 10 to 50 kN/cm.

**3.** The intermediate as claimed in claim 1 or 2, wherein the density of the intermediate is 0.8 to 1.3 g/cm$^3$, preferably 0.9 to 1.20 g/cm$^3$.

**4.** The intermediate as claimed in any one of claims 1 to 3, wherein crystalline cinacalcet or a pharmaceutically acceptable salt thereof with a specific surface area of 0.01 to 8 m$^2$/g is used.

**5.** The intermediate as claimed in any one of claims 1 to 4, wherein hydrophilic polymers with a weight-average molecular weight of less than 150,000 g/mol are used as the hydrophilising agent.

**6.** The intermediate as claimed in claim 5, wherein polyvinyl pyrrolidone, a copolymer of vinyl pyrrolidone and vinyl acetate and/or polyethylene glycol is used as the hydrophilising agent.

**7.** The intermediate as claimed in claim 1, wherein the weight ratio of component (i) to component (ii) is 1:5 to 5:1.

**8.** An immediate-release tablet containing
(α) an intermediate in accordance with claim 1 to 7 and
(β) pharmaceutical excipients.

**9.** The tablet as claimed in claim 8, **characterised in that** component (β) contains disintegrant.

**10.** The tablet as claimed in claim 9, wherein the disintegrant is present in an amount of 10 to 30% by weight, based on the total weight of the formulation.

**11.** The tablet as claimed in any one of claims 8 to 10, wherein the tablet has a cinacalcet content of 40 to 60% by weight.

**12.** Method of preparing a tablet as claimed in any one of claims 8 to 11 comprising the steps of

(a) mixing (i) crystalline cinacalcet or its pharmaceutically acceptable salts with (ii) a hydrophilising agent and optionally further pharmaceutical excipients;
(b) compacting it into flakes;
(c) granulating the flakes;
(d) compressing the resulting granules into tablets, optionally with the addition of further pharmaceutical excipients; and
(e) optionally film-coating the tablets.

**13.** Use of a pH adjuster which is suitable for adjusting a pH value of 3.5 to 5.5, preferably 4 to 5, for preparing a pharmaceutical formulation containing cinacalcet as the active agent.

## Revendications

**1.** Produit intermédiaire que l'on peut obtenir par compactage de

(i) cinacalcet cristallin ou d'un sel de ce dernier, compatible du point de vue pharmaceutique
(ii) d'un produit d'hydrophilisation.

**2.** Produit intermédiaire selon la revendication 1, le compactage étant réalisé dans un compacteur à cylindres et la force de laminage étant de 5 à 70 kN/cm, de préférence de 10 à 50 kN/cm.

**3.** Produit intermédiaire selon la revendication 1 ou 2, la densité du produit intermédiaire étant de 0,8 à 1,3 g/cm$^3$, de préférence de 0,9 à 1,20 g/cm$^3$.

**4.** Produit intermédiaire selon l'une quelconque des revendications 1 à 3, du cinacalcet cristallin ou un sel de ce dernier, compatible du point de vue pharmaceutique étant mis en oeuvre avec une surface spécifique de 0,01 à 8 m$^2$/g.

**5.** Produit intermédiaire selon l'une quelconque des revendications 1 à 4, un polymère hydrophile d'un poids moléculaire moyen inférieur à 150.000 g/mol étant mis en oeuvre en tant que produit d'hydrophilisation.

**6.** Produit intermédiaire selon l'une quelconque des revendications 5, du polyvinylpyrrolidone, un copolymère de vinylpyrrolidone et de vinylacétate et/ou de polyéthylène glycol étant utilisé en tant que produit d'hydrophilisation.

**7.** Produit intermédiaire selon l'une quelconque des revendications 1 à 6, le rapport du poids du composant

(i) au composant (ii) étant de 1 : 5 à 5 : 1.

**8.** Comprimé à libération immédiate, comprenant
($\alpha$) un produit intermédiaire selon l'une quelconque des revendications 1 à 7 et
($\beta$) des produits pharmaceutiques secondaires.

**9.** Comprimé selon la revendication 8, **caractérisé en ce que** le composant ($\beta$) comprend des matières explosives.

**10.** Comprimé selon la revendication 9, la matière explosive étant présente dans une quantité de 10 à 30 % en poids en rapport au poids total de la formulation.

**11.** Comprimé selon l'une quelconque des revendications 8 à 10, le comprimé présentant une teneur en cinacalcet de 40 à 60 % en poids.

**12.** Procédé de production d'un comprimé selon l'une quelconque des revendications 8 à 11, comprenant les étapes :

(a) Mélange (i) de cinacalcet ou de ses sels compatibles du point de vue pharmaceutique avec (ii) un produit d'hydrophilisation ainsi que le cas échéant des produits pharmaceutiques secondaires ;
(b) Compactage en une gale ;
(c) Granulation de la gale;
(d) Compression des granulés qui en résultent en comprimés, le cas échéant en ajoutant des produits pharmaceutiques secondaires supplémentaires ; et
(e) le cas échéant, filmage des comprimés.

**13.** Utilisation d'un adaptateur de pH qui est apte à régler une valeur pH de 3,5 à 5,5, de préférence de 4 à 5, pour la production d'une formulation pharmaceutique qui contient du cinacalcet en tant que principe actif.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1203761 B1
- US 2007185211 A1
- WO 2008027522 A2
- WO 2008000422 A1
- WO 2008064202 A
- WO 2005034928 A
- WO 200762147 A
- WO 200534928 A1